# EUROPEAN PATENT APPLICATION

(11) **EP 3 305 311 A1**
(43) Date of publication of application: **11.04.2018**
(21) Application number: 16800012.3
(22) Date of filing: 24.05.2016
(51) Int. Cl.: A61K 36/82, A61P 3/00, A61P 25/00, A61P 25/28, A61P 43/00

(54) **BRAIN FUNCTION-PROTECTING AGENT**

(30) Priority: 27.05.2015 JP 2015107885
(71) Applicant: Nippon Paper Industries Co., Ltd., Tokyo 114-0002 (JP); Kyushu University National University Corporation, Fukuoka 812-8581 (JP)
(72) Inventor: WASAI, Masafumi, Tokyo 114-0002 (JP); KAWAOKA, Akiyoshi, Tokyo 114-0002 (JP); TACHIBANA, Hirofumi, Fukuoka-shi Fukuoka 812-8581 (JP)
(74) Representative: Müller-Boré & Partner Patentanwälte PartG mbB
(86) International application number: PCT/JP2016/065318
(87) International publication number: WO 2016/190307

(57) **Abstract**

An object is to find out a safe component that can effectively exhibit action including the protection of a brain function, the prevention or alleviation of a reduction in cognitive ability, and the prevention or alleviation of dementia. A component of a plant body of a hybrid between Camellia sinensis and Camellia taliensis is contained as an active component. The hybrid between Camellia sinensis and Camellia taliensis is preferably Sunrouge. A tea leaf extract of the plant body of the hybrid between Camellia sinensis and Camellia taliensis is preferably contained.

SEQUENCE LISTING

<110>  NIPPON PAPER INDUSTRIES CO., LTD.
       KYUSHU UNIVERSITY, NATIONAL UNIVERSITY CORPORATION

<120>  Brain protecting agent

<130>  PNSA-28762

<150>  JP2015-107885
<151>  2015-05-27

<160>  8

<170>  PatentIn version 3.5

<210>  1
<211>  20
<212>  DNA
<213>  Artificial Sequence

<220>
<223>  BACE1 forward primer

<400>  1
agggcttgca cctgtaggac                                                   20


<210>  2
<211>  20
<212>  DNA
<213>  Artificial Sequence

<220>
<223>  BACE1 reverse primer

<400>  2
gcctgagtat gacgccagta                                                   20


<210>  3
<211>  25
<212>  DNA
<213>  Artificial Sequence

<220>
<223>  beta actin forward primer

<400>  3
catccgtaaa gacctctatg ccaac                                             25


<210>  4
<211>  19
<212>  DNA
<213>  Artificial Sequence

<220>
<223>  beta actin reverse primer

<400>  4
atggagccac cgatccaca                                                    19

<210>  5
<211>  19
<212>  DNA
<213>  Artificial Sequence

<220>
<223>  BCL-2 forward primer

<400>  5
cattgtcggg cccatgaag                                                    19


<210>  6
<211>  20
<212>  DNA
<213>  Artificial Sequence

<220>
<223>  BCL-2 reverse primer

<400>  6
caccctggcc caatctagga                                                   20


<210>  7
<211>  22
<212>  DNA
<213>  Artificial Sequence

<220>
<223>  Neprilysin forward primer

<400>  7
ccaaacttaa gcctattctt ac                                                22


<210>  8
<211>  20
<212>  DNA
<213>  Artificial Sequence

<220>
<223>  Neprilysin reverse primer

<400>  8
ccattatgaa cctccaggac                                                   20

## Description

### TECHNICAL FIELD

The present invention relates to a brain function-protecting agent.

### BACKGROUND ART

In recent years, prolonging healthy life expectancy in an aging society is an important challenge in society. Although dementia appears caused by a reduction in a brain function along with aging, relation with lifestyle has been pointed out for Alzheimer-type dementia and cerebrovascular dementia that account for most of dementia.

Disclosed are a brain function-improving agent containing phosphatidyl choline or phosphatidyl serine as an active component (Patent Literature 1), a treating agent containing a salt of deferoxamine as an active component (Patent Literature 2), and a brain function improving composition containing an ester, an amide, a triglyceride, a phosphatide derivative, or the like of a docosahexaenoic acid derivative as an active component (Patent Literature 3) as treating agents for Alzheimer's disease, for example. However, it is difficult to say that these agents can be ingested daily by humans.

On the other hand, catechins as a principal component of tea are reported to exhibit antioxidation action in living bodied and are expected to have the effect of preventing or alleviating a reduction in cognitive function. Reported are that when a mouse is caused to ingest docosahexaenoic acid and/or catechin, memorizing and learning abilities are enhanced (Non Patent Literature 1) and that people who have a habit of frequently drinking green tea can lessen the reduction in cognitive function and have a lower risk of developing dementia than people who do not have a habit of drinking it based on a human observation test (Non Patent Literature 2), for example.

### CITATION LIST

### Patent Literature

Patent Literature 1: Japanese Patent Application Laid-open No. H07-017855
Patent Literature 2: Japanese Patent Application Laid-open No. S58-121213
Patent Literature 3: Japanese Patent Application Laid-open No. H02-049723

Non Patent Literature 1: Ann. Nutr. Metab. Vol. 48, pp. 51-58, 2004
Non Patent Literature 2: PLoS One. 2014 May 14; 9(5): e96013

### SUMMARY OF INVENTION

### TECHNICAL PROBLEM

However, no satisfactory brain function-protecting agent, or no satisfactory agent to prevent a reduction in cognitive ability or improving cognitive ability, has been obtained. An object of the present invention is to find out a safe component that can effectively exhibit action including the protection of a brain function, the prevention or alleviation of a reduction in cognitive ability, and the prevention or alleviation of dementia.

### SOLUTION TO PROBLEM

The inventors of the present invention have found out action to prevent a reduction in cognitive ability in a component of a plant body of a hybrid between Camellia sinensis as a tea plant and Camellia taliensis as a closely related species thereof to reach the present invention.

That is, the present invention provides the following:
[1] A brain function-protecting agent comprising a component of a plant body of a hybrid between Camellia sinensis and Camellia taliensis as an active component.
[2] The agent according to [1], wherein the agent is for alleviating a reduction in cognitive ability, preventing or alleviating dementia, or cranial nerve protection.
[3] The agent according to [1] or [2], wherein the agent regulates one or more selected from the group consisting of accumulation of β-amyloid, BACE expression, BCL expression, and neprilysin expression.
[4] The agent according to [2] or [3], wherein the dementia is Alzheimer-type dementia.
[5] The agent according to any one of [1] to [4], wherein the hybrid between Camellia sinensis and Camellia taliensis is Sunrouge.
[6] The agent according to any one of [1] to [5] comprising a tea leaf extract of the plant body of the hybrid between Camellia sinensis and Camellia taliensis.
[7] A method for protecting a brain function of an object, the method comprising administering an effective amount of an agent containing a component of a plant body of a hybrid between Camellia sinensis and Camellia taliensis as an active component to the object.
[8] Use of a component of a plant body of a hybrid between Camellia sinensis and Camellia taliensis in manufacturing a brain function-protecting agent.

The present invention also provides the following modes:
[1-1] A brain function-protecting agent comprising a component of a plant body of a hybrid between Camellia sinensis and Camellia taliensis as an active component.
[1-2] An agent for preventing or alleviating a reduction in cognitive ability comprising a component of a plant body of a hybrid between Camellia sinensis and Camellia taliensis as an active component.
[1-3] An agent for preventing or alleviating dementia comprising a component of a plant body of a hybrid between Camellia sinensis and Camellia taliensis as an active component.
[1-4] An agent for preventing or alleviating Alzheimer-type dementia comprising a component of a plant body of a hybrid between Camellia sinensis and Camellia taliensis as an active component.
[1-5] A cranial nerve-protecting agent comprising a component of a plant body of a hybrid between Camellia sinensis and Camellia taliensis as an active component.
[1-6] An agent for preventing or alleviating β-amyloid accumulation comprising a component of a plant body of a hybrid between Camellia sinensis and Camellia taliensis as an active component.
[1-7] A BACE expression regulator comprising a component of a plant body of a hybrid between Camellia sinensis and Camellia taliensis as an active component.
[1-8] A BCL expression regulator comprising a component of a plant body of a hybrid between Camellia sinensis and Camellia taliensis as an active component.
[1-9] An agent according to any one of [1-1] to [1-8], in which the hybrid between Camellia sinensis and Camellia taliensis is Sunrouge.
[1-10] An agent according to any one of [1-1] to [1-8] comprising a tea leaf extract of the plant body of the hybrid between Camellia sinensis and Camellia taliensis.
[2-1] A β-amyloid decomposition promoter comprising a component of a plant body of a hybrid between Camellia sinensis and Camellia taliensis as an active component.
[2-2] A neprilysin expression regulator comprising a component of a plant body of a hybrid between Camellia sinensis and Camellia taliensis as an active component.
[2-3] An agent according to [2-1] or [2-2], in which the hybrid between Camellia sinensis and Camellia taliensis is Sunrouge.
[2-4] An agent according to any one of [2-1] to [2-3] comprising a tea leaf extract of the plant body of the hybrid between Camellia sinensis and Camellia taliensis.

### ADVANTAGEOUS EFFECTS OF INVENTION

The present invention provides a brain function-protecting agent, an agent for preventing or alleviating a reduction in cognitive ability, and an agent for preventing or alleviating dementia. The agent of the present invention comprising a component of a plant body of a hybrid between Camellia sinensis and Camellia taliensis as an active component is high in safety even when being ingested for a long period and can be daily ingested.

### BRIEF DESCRIPTION OF DRAWINGS

FIG. 1 is a graph of an evaluation of memory in Test Example 1.
FIG. 2 is a graph of an evaluation of spatial recognition ability and learning ability in Test Example 1.
FIG. 3 is a graph of quantitative results of β-amyloid in extracted mouse brains in Test Example 1.
FIG. 4 is a graph of quantitative results of a BACE1 expression amount in the extracted mouse brains in Test Example 1.
FIG. 5 is a graph of quantitative results of a BCL-2 expression amount in the extracted mouse brains in Test Example 1.
FIG. 6 is a graph of quantitative results of a neprilysin expression amount in Example 2 and Comparative Examples 4 to 6.
FIG. 7 is a graph of quantitative results of a BACE1 expression amount in Example 3 and Comparative Example 7.
FIG. 8 is a graph of quantitative results of β-amyloid in Example 4 and Comparative Example 8.

### DESCRIPTION OF EMBODIMENTS

An agent of the present invention contains a component of a plant body of a hybrid between Camellia sinensis and Camellia taliensis as an active component.

The hybrid between Camellia sinensis and Camellia taliensis is a species obtained by bleeding a plant body belonging to Camellia sinensis and a plant body belonging to Camellia taliensis. The bleeding may be artificial bleeding or natural bleeding such as self-pollination. The hybrid may be either first generation (a first filial hybrid) or its descendant (a natural hybrid between the first filial hybrids, for example). Camellia sinensis is also called a tea plant. Camellia sinensis is preferably a species for green tea (Yabukita, Okumusashi, or the like) and is more preferably Okumusashi. Camellia taliensis is a Theaceae plant, is a closely related species of Camellia sinensis, and is also called akame.

The hybrid between Camellia taliensis and Camellia taliensis is preferably, as a hybrid, "Sunrouge" (Camellia sinensis (L.) Kuntze sp Sunrouge) (the Ministry of Agriculture, Forestry and Fisheries, species registration number: 21262) or F95181 (Cha Chuukanbohon Nou 6) and more preferably "Sunrouge."

Examples of the agent of the present invention include brain function-protecting agents, agents for preventing or alleviating a reduction in cognitive ability, agents for preventing or alleviating dementia, agents for preventing or alleviating Alzheimer-type dementia, cranial nerve-protecting agents, agents for preventing or alleviating β-amyloid accumulation, BACE expression regulators (BACE1 expression inhibitors), BCL expression regulators (BCL-2 expression promoters), β-amyloid decomposition promoters, and neprilysin expression regulators (neprilysin expression promoters).

The agent of the present invention can protect a brain function.

Protecting the brain function includes lessening a reduction in the brain function from a normal state and lessening a further reduction from the brain function that has reduced from the normal state.

The fact that the brain function is being protected can be evaluated by the fact that the reduction in cognitive ability is being prevented or alleviated, measuring the amount of substances related to a reduction in the brain function (e.g., substances related to the reduction in cognitive ability or the appearance of dementia described below), a degree with which β-amyloid is being accumulated in an object's brain, measuring an indicator substance that can predict the degree of accumulation of β-amyloid in the brain, and the fact that cranial nerves are being protected, for example.

Examples of the indicator that can predict the degree of accumulation of β-amyloid in the brain include an Aβ42 value, a total tau value, and a phosphorylated tau value within cerebrospinal fluid.

A method for evaluating the fact that the reduction in cognitive ability is being prevented or alleviated and the fact that cranial nerves are being protected will be described below in detail.

The agent of the present invention can prevent or alleviate the reduction in cognitive ability.

Preventing the reduction in cognitive ability includes maintaining cognitive ability, lessening a further reduction in cognitive ability that has morbidly reduced, and lessening a further reduction in cognitive ability that has reduced within a normal range with age.

Alleviating cognitive ability includes improving cognitive ability that has reduced morbidly or within a normal range.

Examples of cognitive ability include memory, understanding, decision, concentration, and orientation. The agent of the present invention may be one that prevents or alleviates a reduction in partial cognitive ability or one that prevents or alleviates a reduction in the entire cognitive ability.

Cognitive ability can be evaluated by publicly known and used methods. The method of evaluation is not limited; examples thereof include psychological examination, brain wave measurement, MRI, and dementia diagnostic tests. Cognitive ability in mice can be evaluated by a new object recognition test or a Morris water maze test, for example.

The agent of the present invention can prevent or alleviate dementia. Preventing dementia includes lessening affection. Being affected by dementia can be diagnosed in accordance with International Statistical Classification of Diseases and Related Problems, 10th Revision: ICD-10 by World Health Organization, Diagnostic and Statistical Manual of Mental Disorders, Third Edition, Revised: DSM-III-R, or DSM Fourth Edition, Text Revision: DSM-IV-TR by the American Psychiatric Association, for example. Lessening affection includes delaying the progress of the reduction in cognitive ability and maintaining cognitive ability or improving cognitive ability of people whose cognitive ability has abnormally reduced, although they have not reached dementia and delaying the progress of the reduction in cognitive ability and maintaining cognitive ability or improving cognitive ability of people whose cognitive ability has reduced within a normal range.

Dementia is variously classified by cause or the like; dementia targeted in the present invention is not limited to a particular one. Examples of dementia include Alzheimer's disease, cerebrovascular accidents (such as cerebral infarction, cerebral hemorrhage, and subarachnoid hemorrhage). The agent of the present invention may be used for the alleviation of dementia the cause of which is uncertain. The agent of the present invention can efficiently prevent or alleviate Alzheimer-type dementia.

Alleviating dementia includes alleviating, easing, or lessening the worsening of a symptom, although they have already been affected. The fact that the symptom is being alleviated, eased, or lessened to worsen can be checked by the fact that the cognitive ability of dementia patients is being maintained, the fact that the cognitive ability thereof is being improved, or the fact that the progress of the reduction in cognitive ability thereof is being delayed.

The evaluation of cognitive ability when the fact that dementia is being prevented or alleviated is checked can be based on publicly known and used methods as described above.

The evaluation of the fact that Alzheimer-type dementia is being prevented or alleviated can be based on publicly known and used methods, for example. The fact that Alzheimer-type dementia is being prevented or alleviated can be evaluated by brain image analysis by magnetic resonance imaging (MRI), the analysis of brain blood flow state by single photon emission computer tomography (SPECT), brain image analysis by positron emission tomography (PET), the checking of the fact that the accumulation amount of the substances related to the reduction in cognitive ability or the appearance of dementia (e.g., β-amyloid, β-amyloid precursors, hereinafter the same) is being decreased, the checking of the fact that the rate of increase in the accumulation amount of the substances related to the reduction in cognitive ability or the appearance of dementia is being decreased (including the checking of the fact that decomposition is being promoted, hereinafter the same), the checking of the fact that the amount of indicator substances that can predict the degree of accumulation of the substances related to the reduction in cognitive ability or the appearance of dementia is being changed, a decrease in substances related to the promotion of generation of the substances related to the reduction in cognitive ability or the appearance of dementia (e.g., BACE such as BACE1 or BACE2, hereinafter the same), an increase in the expression of substances related to the regeneration of cranial nerves (e.g., BCL such as BCL-2, hereinafter the same), and the checking of an increase in the expression of substances related to the decomposition of the substances related to the reduction in cognitive ability or the appearance of dementia (e.g., neprilysin, hereinafter the same), for example.

The agent of the present invention can protect cranial nerves. Protecting cranial nerves includes maintaining cranial nerves and lessening the dying out of part or the whole of cranial nerve tissue.

The evaluation of the fact that cranial nerves are being protected can be based on publicly known and used methods, for example. The fact that cranial nerves are being protected can be evaluated by the checking of the fact that the accumulation amount of substances involved in damage to cranial nerves (e.g., β-amyloid, β-amyloid precursors, hereinafter the same) is being decreased, the checking of the fact that the rate of increase in the accumulation amount of the substances involved in damage to cranial nerves is being decreased, measuring indicator substances that can predict the degree of accumulation of the substances involved in damage to cranial nerves, a decrease in substances that promote the generation of the substances related to damage to cranial nerves (e.g., BACE such as BACE1 or BACE2, hereinafter the same), an increase in the expression of substances involved in the regeneration of cranial nerves (e.g., BCL such as BCL-2, hereinafter the same), and the checking of an increase in the expression of substances related to the decomposition of the substances related to damage to cranial nerves (e.g., neprilysin, hereinafter the same), for example.

The agent of the present invention can prevent or alleviate the accumulation of β-amyloid. Preventing or alleviating accumulation includes decreasing the accumulation amount, maintaining the accumulation amount, decreasing the rate of increase in the accumulation amount, and promoting the decomposition of the accumulated β-amyloid. The evaluation of the fact that the accumulation of β-amyloid is being prevented or alleviated can be based on publicly known and used methods, for example; the fact that the accumulation of β-amyloid is being prevented or alleviated can be evaluated by the checking of the fact that the accumulation amount of β-amyloid in the brain is being decreased, the checking of the fact that the rate of increase in the accumulation amount is being decreased, and measuring indicator substances that can predict the degree of accumulation of β-amyloid in the brain, for example. Examples of the indicator substances include substances that promote the generation of β-amyloid (e.g., BACE such as BACE1 or BACE2, hereinafter the same) and substances related to the decomposition of β-amyloid (e.g., neprilysin, hereinafter the same).

The agent of the present invention can promote the decomposition of β-amyloid. The agent of the present invention promotes the decomposition of β-amyloid, thereby preventing or alleviating the accumulation of β-amyloid in the brain and preventing or alleviating dementia (e.g., Alzheimer-type dementia). The evaluation of the fact that the decomposition of β-amyloid is being promoted can be based on publicly known and used methods, for example; the fact that the decomposition of β-amyloid is being promoted can be evaluated by measuring the amount of the substances related to the decomposition of β-amyloid, for example.

Objects to which the agent of the present invention is administered are not limited to particular objects and may be animals other than humans; examples thereof include pets (dogs, cats, and parakeets), domestic animals (cows, pigs, goats, sheep, and horses), domestic fowls (chickens, quails, and turkeys), and experimental animals (mice, rats, hamsters, marmots, and rabbits).

The health state of the objects of the agent of the present invention is not limited to a particular state; the objects may be any of people who maintain cognitive ability, people whose cognitive ability has reduced within a normal range, people with suspected dementia, dementia patients, people whose brain function is normally maintained, people whose brain function has reduced within a normal range, people whose brain function has abnormally reduced, people whose β-amyloid accumulation amount in the brain is within a normal range or abnormal, people whose accumulation amount of a substance related to dementia or cognitive ability is within a normal range or abnormal, people whose expression amount of a substance selected from a substance related to the promotion of generation of the substance, a substance related to the decomposition of the substance, and a substance related to the regeneration of cranial nerves is within a normal range or abnormal, and the like. The age of the objects is also not limited to a particular age; the objects may be any of minors, adults, the elderly, and the like, are preferably adults, and are more preferably the elderly. The objects are not limited to humans and may be animals other than humans. Examples of the dementia patients or people with suspected dementia include patients of Alzheimer-type dementia or people with suspected Alzheimer-type dementia and patients of vascular dementia or people with suspected vascular disease dementia. The objects of the agent of the present invention are preferably dementia patients or people with suspected dementia and are more preferably patients of Alzheimer-type dementia or people with suspected Alzheimer-type dementia.

The component of the plant body may be a component contained in at least a part of the plant body. Examples of the part of the plant body include roots, stems, leaves, flowers, fruits, branches, seeds, and sprouts. Among these parts, a part containing stems and/or leaves is preferred, and stems and/or leaves are more preferred. Examples of a method for preparing the component of the plant body of the hybrid between Camellia sinensis and Camellia taliensis include, but are not limited to, extraction from a crushed body of the plant body or the plant body and purification. The agent of the present invention preferably contains the component of the plant body of the hybrid between Camellia sinensis and Camellia taliensis as pulverized product from leaves of the plant body or a leave extract of the plant body.

The agent of the present invention contains the component of the plant body of the hybrid between Camellia sinensis and Camellia taliensis as an active component. Examples of the component of the plant body of the hybrid between Camellia sinensis and Camellia taliensis that may be contained in the agent of the present invention include polyphenols, anthocyanin, delphinidin, glycosides of delphinidin, catechin type compounds (epigallocatechin gallate, for example), caffeine, amino acids, salts of amino acids, hydrolyzable tannin (strictinin, for example), theogallin, and theogallin derivatives.

In the present specification, the catechin type compounds mean compounds with 3-hydroxy-2-phenylchromane as a nucleus and hydrogen atom(s) of the nucleus replaced with one or more hydroxy groups. When the agent of the present invention contains the catechin type compounds, one catechin type compound may be contained, or two or more of them may be contained.

The component may be one component alone or a combination of two or more components. The agent of the present invention preferably contains the component of the plant body of the hybrid between Camellia sinensis and Camellia taliensis as an extract of the plant body. The agent of the present invention may contain one or two or more components separated from the extract of the plant body as the component of the plant body of the hybrid between Camellia sinensis and Camellia taliensis. The agent of the present invention may contain the one or two or more components separated from the extract of the plant body in addition to the extract of the plant body.

Examples of polyphenols that may be contained in the agent of the present invention include flavonoids (catechin, anthocyanin, tannin, rutin, and isoflavone), phenolic acid, ellagic acid, lignan, curcumin, coumarin, and unidentified polyphenols.

The total content of polyphenols of the agent of the present invention is preferably 100.0% by weight or less, more preferably 90.0% by weight or less, and further preferably 80.0% by weight or less relative to the dry weight of the active component (when the agent of the present invention contains a tea leaf extract, the tea leaf extract).

The content of polyphenols can be measured by high-performance liquid chromatography (HPLC), the Folin-Ciocalteu method, or colorimetry.

The agent of the present invention preferably contains at least one catechin type compound as the component of the plant body of the hybrid between Camellia sinensis and Camellia taliensis and preferably contains at least one catechin type compound and one or more selected from caffeine, amino acids, salts of amino acids, hydrolyzable tannin, theogallin, and theogallin derivatives. When the agent of the present invention contains two or more components, the agent of the present invention can be renamed a composition.

Examples of the catechin type compounds include catechin (C), gallocatechin (GC), catechin gallate (CG), gallocatechin gallate (GCG), epicatechin (EC), epigallocatechin (EGC), epicatechin gallate (ECG) and epigallocatechin gallate (EGCG), epigallocatechin-3-(3"-O-methyl)gallate (EGCG3"Me), and epicatechin-3-(3"-O-methyl)gallate (ECG3"Me).

The content of the catechin type compounds of the agent of the present invention is preferably 50.0% by weight or less, more preferably 40.0% by weight or less, further preferably 35.0% by weight or less, and still more preferably 30.0% by weight or less or 25.0% by weight or less relative to the dry weight of the active component (when the agent of the present invention contains the tea leaf extract, the tea leaf extract). The lower limit thereof, which is not limited to a particular value, is usually 14.0% by weight or more. When the content is within this range, bitterness and astringency are less intense, giving excellent palatability.

When the agent of the present invention contains epigallocatechin gallate (EGCG) among the catechin type compounds, the content of EGCG is preferably 30.0% by weight or less, more preferably 20.0% by weight or less, and further preferably 15.0% by weight or less relative to the dry weight of the active component (when the agent of the present invention contains the tea leaf extract, the tea leaf extract).

The content of the catechin type compounds can be measured by HPLC or colorimetry.

Caffeine is one kind of purine alkaloids having a purine ring. Caffeine is contained in food and drink such as coffees, colas, green teas, black teas, oolong teas, cocoas, chocolates, and nutritional drinks; caffeine contained in teas bonds to tannin, and its effect is lessened, whereby stimulant action like coffee acts weakly and slowly.

The content of caffeine of the agent of the present invention is preferably 5.4% by weight or less, more preferably 5.2% by weight or less, and further preferably 5.1% by weight or less relative to the dry weight of the active component (when the agent of the present invention contains the tea leaf extract, the tea leaf extract). The lower limit thereof, which is not limited to a particular value, is usually 2.5% by weight or more when low caffeine treatment is not performed.

The content of caffeine can be measured by HPLC.

Amino acids are compounds having both an amino group and a carboxyl group. Examples of amino acids include theanine, glycine, arginine, lysine, alanine, glutamine, glutamic acid, histidine, threonine, asparagine, aspartic acid, phenylalanine, leucine, valine, tryptophan, proline, cysteine, serine, tyrosine, isoleucine, and methionine. Examples of salts of amino acids include alkali metal salts, alkali earth metal salts, ammonium salts, inorganic acid salts, and organic acid salts.

The content of amino acids or salts thereof of the agent of the present invention is preferably 1.0% by weight or more, preferably 1.3% by weight or more, and more preferably 1.5% by weight or more relative to the dry weight of the active component (when the agent of the present invention contains the tea leaf extract, the tea leaf extract). The upper limit thereof, which is not limited to a particular value, is usually 4.5% by weight or less.

The content of amino acids or salts thereof relative to the dry weight of the agent of the present invention can be measured by HPLC.

The agent of the present invention preferably contains hydrolyzable tannin. Arising from containing hydrolyzable tannin, the agent of the present invention can exhibit an antiallergic effect. Examples of hydrolyzable tannin include strictinin, which is preferred. Strictinin is 1,2-di-O-galloyl-4,6-O-(S)-hexahydroxydiphenoyl-β-D-glucopyranose.

The content of hydrolyzable tannin of the agent of the present invention is preferably 5.0% by weight or less, more preferably 1.5% by weight or less, and further preferably 3.5% by weight or less relative to the dry weight of the active component (when the agent of the present invention contains the tea leaf extract, the tea leaf extract). The lower limit thereof, which is not limited to a particular value, is usually 0.6% by weight or more.

The content of hydrolyzable tannin such as strictinin can be measured by HPLC.

The agent of the present invention preferably contains theogallin or derivatives thereof. Arising from containing theogallin or derivatives thereof, an antiallergic effect can be obtained. Theogallin is a glycoside of trihydroxy benzoic acid and is also called (1S)-1β,3β,4β-trihydroxy-5α-(galloyloxy)cyclohexane carboxylic acid.

The content of theogallin or derivatives thereof is preferably 10.0% by weight or less, more preferably 3.0% by weight or less, and further preferably 1.6% by weight or less relative to the dry weight of the active component (when the agent of the present invention contains the tea leaf extract, the tea leaf extract). The lower limit thereof, which is not limited to a particular value, is usually 0.7% by weight or more.

The content of theogallin can be measured by HPLC.

The agent of the present invention preferably contains tea leaves of the plant body of the hybrid between Camellia sinensis and Camellia taliensis, more preferably contains the tea leaf extract, and further preferably contains the tea leaf extract as an active component. With this composition, the active component can be contained in a balanced manner, and various kinds of effects can be markedly exhibited.

The extraction condition of the tea leaf extract is not limited to a particular condition; the tea leaf extract can be obtained by extracting tea leaves of a hybrid between Camellia sinensis and Camellia taliensis, for example.

The tea leaves are usually subjected to tea manufacturing before extraction. Examples of manufactured tea leaves include green tea, black tea, and oolong tea; green tea is preferred. Examples of tea leaves of green tea include ordinary green tea, refined green tea, coarse green tea, twig tea, bud tea, powdered green tea, roasted tea, coarse green tea mixed with roasted brown rice, steamed and dried tea, pan-roasted tea, and paochung tea.

At least part of the tea leaves may be subjected to crushing treatment before extraction. For the crushing treatment, crushers (MULTI-BEADS-SHOCKER (registered trademark, Yasui Kikai Corporation), for example), stone mills, ceramic mills, ball mills, hammer mills, or the like can be used.

Examples of an extractant include water and aqueous ethanol solution; water is preferred. The concentration of ethanol of the aqueous ethanol solution can be determined to the extent that the effect of the present invention is not impaired, which is usually 70% by weight or less relative to the entire aqueous ethanol solution. When extraction is performed using water, the extraction temperature is preferably 30 to 150°C. The extraction time is preferably 1 to 60 minutes. As to the weight ratio of water and the tea leaves, preferably used is water at least 10 times heavier than the dry weight of the tea leaves, and more preferably used is water 10 to 300 times heavier than the dry weight of the tea leaves relative to the tea leaves of 1. When extraction is performed with an organic solvent such as acetic acid, the content of catechins (the catechin type compounds) is high, although extraction efficiency is good, which may give strong bitterness and astringency and may worsen palatability. Stirring may be performed during extraction.

After the extraction treatment, solid content may be removed (solid-liquid separated) by treatment such as filtration, centrifugation, or sifting. Filtration may be performed once or two or more times. During filtration, a means such as filter paper or a line filter can be used. Centrifugation can be performed at 500 to 10,000 G, and a time therefor can be 1 to 60 minutes. Further, treatment such as concentration (such as vacuum concentration or reverse osmosis membrane treatment) or drying (such as spray drying or freeze drying) may be performed. These treatments may be selected in accordance with the form of the agent.

The form of the agent of the present invention is not limited to a particular form. Examples of the form include liquid (such as capsules and soft capsules), slurry (such as syrups), semi-solid (such as creams and pastes), and solid (such as tablets and powder (such as granules and fine grains)); the form is preferably solid and is preferably powder. The dosage form of the agent of the present invention is not limited; examples of the dosage form include pills, tablets, granules, suspensions, emulsions, powders, syrups, and troches.

The administration form of the agent of the present invention is not limited to a particular form; examples thereof include oral administration, parenteral administration (such as transpulmonary administration, subcutaneous administration, intravenous administration, intraspinal administration, and dermal administration), sublingual administration, and inhalation administration; the administration form is usually oral administration.

The administration time of the agent of the present invention is not limited to a particular time. The administration time may be after rising, before meals (from 30 minutes before meals to the start of meals, for example), after meals (from the end of meals to 30 minutes after meals, for example), between meals (two or three hours after the end of meals, for example), before going to bed, or simultaneously with meals, for example.

The agent of the present invention may contain components other than the active component. When the agent of the present invention contains the components other than the active component, the agent of the present invention may be renamed a composition. Examples of the components other than the active component include various kinds of additives. Specific examples of the additives include the following: seasonings; acidulants (such as citric acid and succinic acid); preservatives (such as ascorbic acid, acetates, and ε-polylysine); pH regulators; emulsifiers (such as sucrose fatty acid esters, glycerin fatty acid esters, organic acid monoglycerides, and lecithin); flavorings; dyes; thickeners (such as carrageenan and xanthan gum); inflating agents; proteins (such as soybean proteins and milk proteins); saccharides (such as starch, sucrose, fructose, reduced starch saccharified products, erythritol, and xylitol); sweetening agents (such as sucralose and thaumatin); vitamins (such as vitamin A, vitamin C, vitamin E, and vitamin K); and minerals (such as iron and calcium).

The agent of the present invention is available as food and drink or food and drink additives. Examples of the food and drink include drinks (such as soft drinks, carbonated drinks, nutritional drinks, powdered drinks, fruit drinks, lactic drinks, jelly drinks, beers, sakes, foreign liquors, Chinese liquors, and alcoholic medical drinks); cooked rices (such as cooked rice and rice porridge); bread goods; seasonings (such as sauces, misos, soy sources, mayonnaises, shortenings, dressings, soy-based sauces, and spices); processed-soybean products (such as Nattos (fermented soybeans), Tofus (soybean curds), and aburaages (deep-fried tofus)); processed marine products (such as kamabokos (boiled fish pastes), hanpens (cakes of ground fish combined with starch and steamed), chikuwas (boiled fish pastes), and fish-paste products); processed meat products (such as hams, sausages, and vienna sausages); processed agricultural foods (such as vegetables and fruits); tsukemonos (pickles); noodles (such as udons (thick white noodles made from wheat flour), sobas (buckwheat noodles), and spaghettis); soups (such as powdered soups and liquid soups); lactic products (such as cheeses, yogurts, and creams); confectionaries (such as jellies, snack foods, chewing gums, candies, chocolates, and cakes); health foods (functional foods, nutritional supplements (supplements), and foods for specified health uses); and specifically targeted foods and drinks (medical foods, foods for sick people, foods for babies, nursing care foods, and foods for the elderly). Examples of foods and drinks targeted by the food and drink additives are similar to the specified examples. Further, the agent of the present invention is available as feed or feed additives. Feed may be targeted at animals other than humans.

Although the administration time of the agent of the present invention is not limited to a particular time, it is preferably daily ingested.

The administration amount of the agent of the present invention, which is not limited to a particular amount, can be administered as appropriate in accordance with the age, body weight, health state, or the like of an administration object. The agent of the present invention can lessen the reduction in cognitive ability more effectively than an agent containing a component of Camellia sinensis as an active component and can thereby reduce the lower limit of the daily administration amount of the agent of the present invention compared with the agent containing the component of Camellia sinensis as the active component. Consequently, the daily administration amount of the agent of the present invention is preferably 50 mg or more, more preferably 100 mg or more, and further preferably 150 mg or more. The upper limit of the daily administration amount of the agent of the present invention, which is not limited, is usually 10,000 mg or less.

### [Examples]

The following describes the present invention further specifically with reference to examples and comparative examples; these examples do not limit the present invention.

Test Example 1 Evaluation of Effect of Preventing Reduction in Cognitive Ability and Brain Function-Protecting Effect

### [Example 1]

Sunrouge dried tea leaves obtained by harvesting Sunrouge tea leaves grown in 2014 and steaming and drying the tea leaves were used as tea leaves. Distilled water in a volume of 1,400 mL was boiled, and 70 g of the Sunrouge dried tea leaves were added thereto. After extraction was performed in the boiling water for 10 minutes, the liquid was filtered using a piece of gauze. The filtrate was centrifuged at 700 G for 10 minutes to obtain supernatant fluid as a tea leave extract. The tea leave extract was treated by a vacuum freeze dryer (the model FZ-12CS with a BTD shelf type drying chamber manufactured by Labconco) to obtain freeze-dried powder. Table 1 lists results of the analyses of the content of catechins (he catechin type compounds) and total polyphenols in the freeze-dried powder of the Sunrouge tea leave extract. The amount of "total catechins" listed in Table 1 means a total amount of the catechin type compounds contained in the sample. The following shows the content of each of various kinds of components in the freeze-dried powder of the Sunrouge tea leaf extract:
Caffeine 5.1% by weight
Amino acids 1.5% by weight
Hydrolyzable tannin 1.4% by weight
Theogallin 1.5% by weight

The obtained freeze-dried powder of the Sunrouge tea leaf extract in an amount of 0.2% by weight was added to feed (AIN-93M manufactured by Oriental Yeast Co., Ltd.) to be mixed therewith. Table 2 lists the feed compositions.

Six male senescence-accelerated model mice SAMP8 (from Japan SLC, Inc.) were used as an administration group of the freeze-fried powder of the Sunrouge tea leaf extract. Fifteen-week-old mice SAMP8 were preliminarily raised for four weeks and were then raised for 21 weeks while being caused to freely ingest the feed with the freeze-dried powder of the Sunrouge tea leaf extract added so as to be 5 grams per day for one mouse. After the end of the raising period, the following behavior analysis tests were conducted.

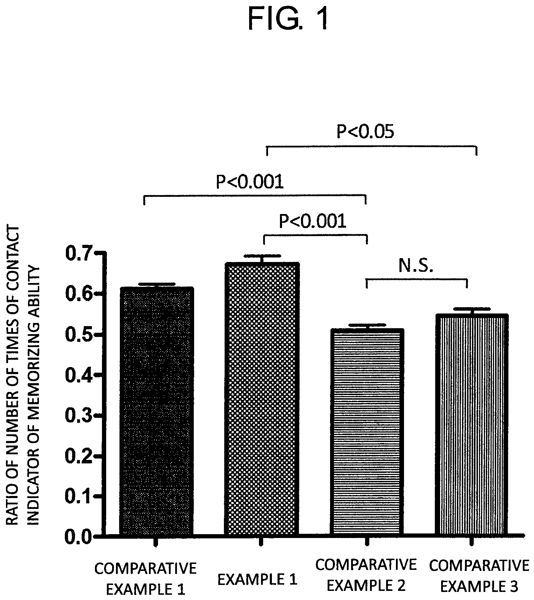

A new object recognition test as an indicator of memory was performed as Behavior Analysis Test (1). The mice were acclimatized to an environment within a transparent, 25 cm x 30 cm raising cage for 2 hours. After that, the two same Objects A and A were put into the cage. The numbers of times of contact with the respective objects were measured for 10 minutes, and Objects A and A were removed after the measurement. Eighteen hours after Objects A and A had been removed, two different Objects A and B were put into the raising cage. The numbers of times of contact with the respective objects were measured for 10 minutes, and the ratio of the number of times of contact with Object B to a total number of times of the number of times of contact with Object A and the number of times of contact with Object B was determined. FIG. 1 illustrates the results. The presence or absence of a significant difference was determined by a Tukey's test, the results of which are illustrated in FIG. 1. Mice with memories having not declined memorize Objects A and A and recognize Object B out of Objects A and B put into the cage after 18 hours as a new object to come into contact with Object B more times than Object A. In contrast, mice with memories having declined forget Objects A and A and recognize both Objects A and B put into the cage after 18 hours as new objects to come into contact with them comparably. Consequently, a higher ratio of the number of times of contact with Object B to a total number of times of the number of times of contact with Object A and the number of times of contact with Object B means a better memory.

A Morris water maze test as an indicator of spatial recognition ability and learning ability was conducted as Behavior Analysis Test (2). Water at 23°C ± 1°C was poured into a circular pool with a diameter of 120 cm × a height of 30 cm and a periphery surrounded to make a scene constant (manufactured by Muromachi Kikai Co., Ltd.) up to a height of 20 cm. The circular pool was divided crosswise into four sections, and the respective quarter sections were named A, B, C, and D. A foothold (manufactured by Muromachi Kikai Co., Ltd.) as a goal with a diameter of 10 cm was installed in Section A so as to have a height of 1 cm below water. The installation environment was photographed with a camera from above and was recorded with behavior analysis software (ANY-maze from Stoelting Co.).

The mice were put into Section B and were caused to swim in the pool for 2 minutes as training. When any of the mice reached the goal within 2 minutes, the mouse was then placed on the goal for 20 seconds to cause it to memorize a surrounding scene. If any of the mice was not able to reach the goal within 2 minutes, the mouse was forced to move to the goal and was then caused to memorize the surrounding scene for 20 seconds. Following Section B, the mice were put into Section C, in which similar operations were conducted. Following Section C, the mice were put into Section D, in which similar operations were conducted. The foregoing operations were training for one day. On the second day, training was conducted in an order of Section C, Section D, and Section B. On the third day, training was conducted in an order of Section D, Section B, and Section C. On the fourth day, training was conducted in an order of Section B, Section C, and Section D.

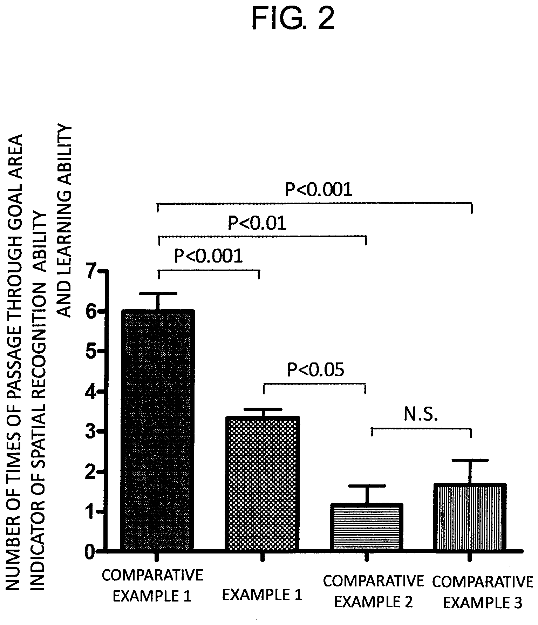

On the fifth day, the foothold as the goal was removed, and the mice were put into Section C positioned diagonally to the goal and were caused to swim in the pool for 2 minutes. The swimming trajectories of the mice were recorded with the behavior analysis software, and the number of times of passage through the area in which the goal had been installed was measured. FIG. 2 illustrates the results. The presence or absence of a significant difference was determined by a Tukey's test, the results of which are illustrated in FIG. 2. Mice the spatial recognition ability and the learning ability of which have not declined memorize the position of the goal and the surrounding scene and continue to search for the goal within the 2 minutes' swimming time to pass through the goal a large number of times. In contrast, mice the spatial recognition ability and the learning ability of which have declined are unable to memorize and learn the position of the goal, and the number of times of passage through the goal within the 2 minutes' swimming time reduces. Consequently, a larger number of times of passage through the goal means higher spatial recognition ability and learning ability.

Proteins of a mouse brain were analyzed by the following procedure. After the end of Behavior Analysis Tests (1) and (2), the mice were abstained from food for 16 hours and were slaughtered, and the brains were extracted. To the extracted brains was added a liquid mixture of autoclave-sterilized Buffer A: 1 ml, 2 µg/mL Aprotinin (manufactured by Sigma-Aldrich): 1 µL, 1 mM phenylmethylsulfonyl fluoride (manufactured by Wako Pure Chemical Industries, Ltd.): 10 µL, and 1 mM pervanadate (manufactured by Wako Pure Chemical Industries, Ltd.): 50 µL, and the mixture was homogenized with a sterilized BioMasher II (manufactured by Nippi, Incorporated). The brain homogenate was centrifuged at 4°C and 12,000 G for 10 minutes, and the supernatant was collected to obtain a protein analysis sample. Table 3 lists the composition of Buffer A.

A total protein amount in the protein analysis sample was measured using Bicinchoninic Acid (BCA) Protein Assay Reagent (manufactured by Life Technologies) and SPECTRA FLUOR (TACAN).

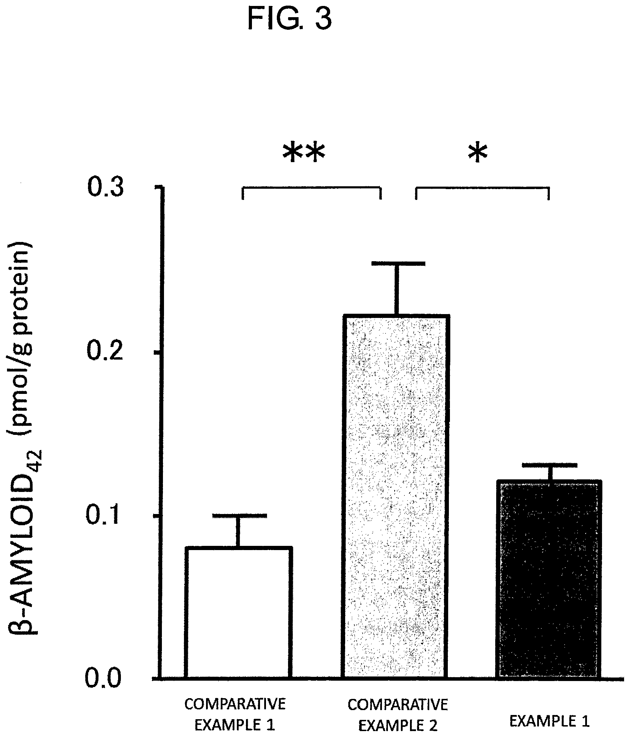

β-amyloid 42 (1-42) in the protein analysis sample was quantified with Human/Rat βAmyloid (42) ELISA kit (manufactured by Wako Pure Chemical Industries, Ltd.). FIG. 3 illustrates the results. The presence or absence of a significant difference was determined by a Tukey's test, the results of which are illustrated in FIG. 3. It is considered that Alzheimer's disease is caused by the β-amyloid protein (Aβ) flocculating in the brain and depositing as senile plaques (amyloid plaques), causing nerve cells to die out in the end. Consequently, a smaller amount of β-amyloid in the brain exhibits a higher effect of lessening Alzheimer-type dementia.

Gene expression in the mouse brain was analyzed by the following procedure. The total RNA of the extracted brains was extracted with PURELINK RNA MINI KIT (manufactured by Life technologies), and a cDNA was synthesized with PrimeScript (registered trademark) RT reagent Kit (manufactured by Takara Bio Inc.) to obtain a gene expression analysis sample.

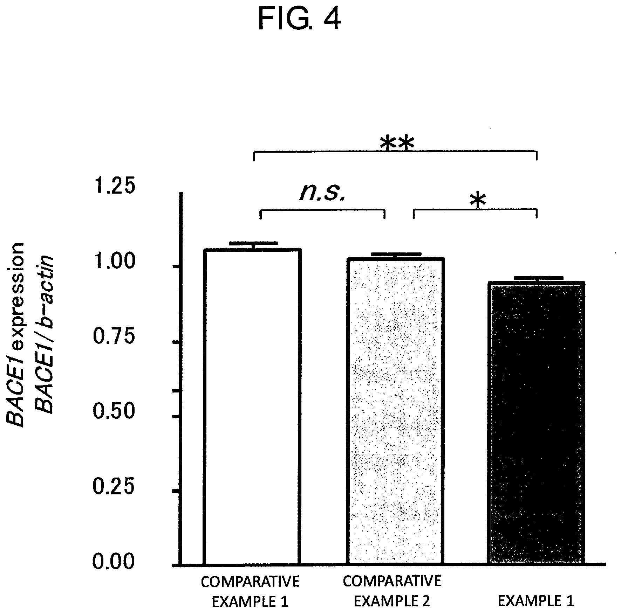

With the cDNA of the gene expression analysis sample as a template, the gene expression amount of a BACE1 gene (forward primer: 5'-AGGGCTTGCACCTGTAGGAC-3': SEQ ID NO: 1 and reverse primer: 5'-GCCTGAGTATGACGCCAGTA-3': SEQ ID NO: 2) was measured by the real-time PCR method using Sso Advanced™ Universal SYBR (registered trademark) Green Supermix (Bio-Rad Laboratories, Inc.) and C1000™ Thermal Cycler (CFX96™ Optics Module, Bio-Rad Laboratories, Inc.). As an internal standard, a β-actin gene (forward primer: 5'-CATCCGTAAAGACCTCTATGCCAAC-3': SEQ ID NO: 3 and reverse primer: 5'-ATGGAGCCACCGATCCACA-3': SEQ ID NO: 4) was used. FIG. 4 illustrates the results. The presence or absence of a significant difference was determined by a Tukey's test, the results of which are illustrated in FIG. 4. BACE1 is an enzyme that cuts the N-terminus of the β-amyloid precursor protein (APP). β-Amyloid cut out of APP by BACE1 has high flocculating property and forms amyloid to cause Alzheimer-type dementia. A smaller BACE1 expression amount further lessens the cut-out of β-amyloid and exhibits a higher effect of lessening Alzheimer-type dementia.

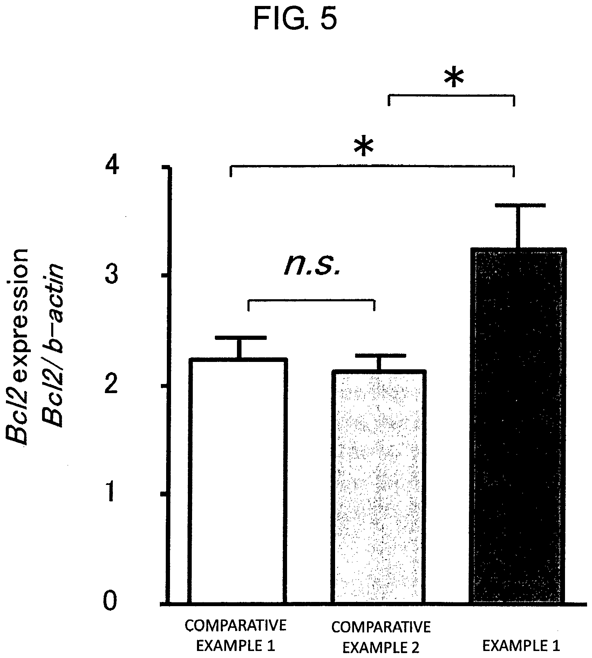

With the cDNA of the gene expression analysis sample as a template, the gene expression amount of a BCL-2 gene (forward primer: 5'-CATTGTCGGGCCCATGAAG-3': SEQ ID NO: 5 and reverse primer: 5'-CACCCTGGCCCAATCTAGGA-3': SEQ ID NO: 6) was measured by the real-time PCR method using Sso Advanced™ Universal SYBR (registered trademark) Green Supermix (Bio-Rad Laboratories, Inc.) and C1000™ Thermal Cycler (CFX96™ Optics Module, Bio-Rad Laboratories, Inc.). As an internal standard, a β-actin gene was used. FIG. 5 illustrates the results. The presence or absence of a significant difference was determined by a Tukey's test, the results of which are illustrated in FIG. 5. BCL-2 is an antiapoptotic protein, and a larger BCL-2 expression amount in the brain exhibits higher nerve protective action and a higher effect of lessening Alzheimer-type dementia.

### [Comparative Example 1]

Six male normal mice SAMR1 (from Japan SLC, Inc.) were used as a positive control group. Fifteen-week-old mice SAMR1 were preliminarily raised for four weeks and were then raised for 21 weeks while being caused to freely ingest feed (AIN-93M manufactured by Oriental Yeast Co., Ltd.) so as to be 5 grams per day for one mouse. Table 2 lists the feed composition. After the end of the raising period, Behavior Analysis Tests (1) and (2) were conducted similarly to Example 1. After the end of the behavior analysis tests, protein analysis and gene expression analysis were conducted similarly to Example 1.

### [Comparative Example 2]

Raising was performed similarly to Comparative Example 1 except that six male senescence-accelerated model mice SAMP8 (from Japan SLC, Inc.) were used as a negative control group. After the end of the raising period, Behavior Analysis Tests (1) and (2) were conducted similarly to Example 1. After the end of the behavior analysis tests, protein analysis and gene expression analysis were conducted similarly to Example 1.

### [Comparative Example 3]

Raising was performed similarly to Examples 1 except that Yabukita dried tea leaves obtained by harvesting Yabukita tea leaves grown in 2014 and steaming and drying the tea leaves were used in place of the Sunrouge dried tea leaves and that six male senescence-accelerated model mice SAMP8 (from Japan SLC, Inc.) were used. After the end of the raising period, Behavior Analysis Tests (1) and (2) were conducted similarly to Example 1. Table 1 lists results obtained by analyzing a freeze-dried powder component of a Yabukita tea leave extract obtained in Comparative Example 3. Table 2 lists the composition of the feed used in Comparative Example 3.

### [Table 1]

**Table 1**

| Measurement method | Measurement item | Unit | Example 1 | Comparative Example 3 |
|---|---|---|---|---|
| Analysis result by Japan Food Research Laboratories | Catechin | g/100g | 0.23 | 0.37 |
| | Epicatechin | g/100g | 1.40 | 2.40 |
| | Gallocatechin | g/100g | 0.87 | 2.60 |
| | Epipallocatechin | g/100g | 5.40 | 12.00 |
| | Epigallocatechin gallate | g/100g | 9.90 | 10.00 |
| | Gallocatechin gallate | g/100g | 0.64 | 1.00 |
| | Epicatechin gallate | g/100g | 1.90 | 1.60 |
| | Catechin gallate | g/100g | 0.06 | 0.08 |
| | Epigallocatechin-3-O-(3-O-methyl)gallate | g/100g | 0.39 | 0.00 |
| | Total catechins | g/100g | 20.79 | 30.05 |
| Folin-Ciocalteu method | Total polyphenols | g/100g | 55.0 | 61.7 |

### [Table 2]

**Table 2**

| | Unit | Comparative Example 1 | Example 1 | Comparative Example 2 | Comparative Example 3 |
|---|---|---|---|---|---|
| Sunrouge F.D. | w/w | - | 0.2% | - | - |
| Yabukita F.D. | w/w | - | - | - | 0.2% |
| Milk casein | g | 14.00000 | 14.00000 | 14.00000 | 14.00000 |
| L-cystine | g | 0.18000 | 0.18000 | 0.18000 | 0.18000 |
| Cornstarch | g | 46.56920 | 46.56920 | 46.56920 | 46.56920 |
| *α*-cornstarch | g | 15.50000 | 15.50000 | 15.50000 | 15.50000 |
| Sucrose | g | 10.00000 | 10.00000 | 10.00000 | 10.00000 |
| Soybean oil | g | 4.00000 | 4.00000 | 4.00000 | 4.00000 |
| Cellulose powder | g | 5.00000 | 5.00000 | 5.00000 | 5.00000 |
| AIN93M Mineral | g | 3.50000 | 3.50000 | 3.50000 | 3.50000 |
| AIN93 Vitamin | g | 1.00000 | 1.00000 | 1.00000 | 1.00000 |
| Choline bitartrate | g | 0.25000 | 0.25000 | 0.25000 | 0.25000 |
| tert-Butyl hydroquinone | g | 0.00080 | 0.00080 | 0.00080 | 0.00080 |

### [Table 3]

**Table 3**

| Reagent | Amount |
|---|---|
| Tris | 50mM |
| NaCl | 150mM |
| EDTA | 1mM |
| NaF | 50mM |
| Na₄P₂O₇ | 30mM |
| Triton-X100 | 1% (V/V) |
| pH | 7.5 (regulated with HCl) |

As listed in Table 1, Sunrouge was lower in total catechins (the catechin type compounds) reported as components involved in dementia prevention than Yabukita. Yabukita and Sunrouge were comparable in epigallocatechin gallate reported as a component involved in dementia prevention. Yabukita and Sunrouge were comparable in total polyphenols.

As illustrated in FIG. 1, the mice of Example 1, which had ingested the Sunrouge tea leaf extract, significantly increased in the rate of contact with the new object compared with the mice of Comparative Example 2, which had not ingested the tea leaf extract, and the mice of Comparative Example 3, which had ingested the Yabukita tea leaf extract. This indicates that the agent of the present invention effectively lessens a reduction in memory.

As illustrated in FIG. 2, the mice of Example 1, which had ingested the Sunrouge tea leaf extract, significantly increased in the number of times of passage through the goal area compared with the mice of Comparative Example 2, which had not ingested the tea leaf extract. The mice of Comparative Example 3, which had ingested the Yabukita tea leaf extract, showed no change in the number of times of passage through the goal area compared with the mice of Comparative Example 2, which had not ingested the tea leaf extract. This indicates that the agent of the present invention effectively lessens reductions in spatial recognition ability and learning ability.

As illustrated in FIG. 3, the mice of Example 1, which had ingested the Sunrouge tea leaf extract, significantly reduced in the amount of β-amyloid compared with the mice of Comparative Example 2, which had not ingested the tea leaf extract. This indicates that the agent of the present invention effectively lessens the accumulation of β-amyloid and also indicates that the agent of the present invention can protect the brain function, can prevent or alleviate Alzheimer-type dementia, and can protect cranial nerves.

As illustrated in FIG. 4, the mice of Example 1, which had ingested the Sunrouge tea leaf extract, were significantly lower in the expression amount of the BACE1 gene than the normal mice of Comparative Example 1 and the mice of Comparative Example 2, which had not ingested the tea leaf extract. This indicates that the agent of the present invention effectively lessens the expression of BACE1 and also indicates that the agent of the present invention can protect the brain function, can prevent or alleviate Alzheimer-type dementia, and can protect cranial nerves.

As illustrated in FIG. 5, the mice of Example 1, which had ingested the Sunrouge tea leaf extract, were significantly higher in the expression amount of the BCL-2 gene than the normal mice of Comparative Example 1 and the mice of Comparative Example 2, which had not ingested the tea leaf extract. This indicates that the agent of the present invention effectively promotes the expression of BCL-2 and also indicates that the agent of the present invention can protect the brain function, can prevent or alleviate Alzheimer-type dementia, and can protect cranial nerves.

The foregoing results of the example indicate that the agent of the present invention can lessen the reduction in cognitive ability and can protect the brain function. These results also indicate that the agent of the present invention can exhibit an effect as an agent to prevent or alleviate dementia (especially Alzheimer-type dementia).

### [Example 2]

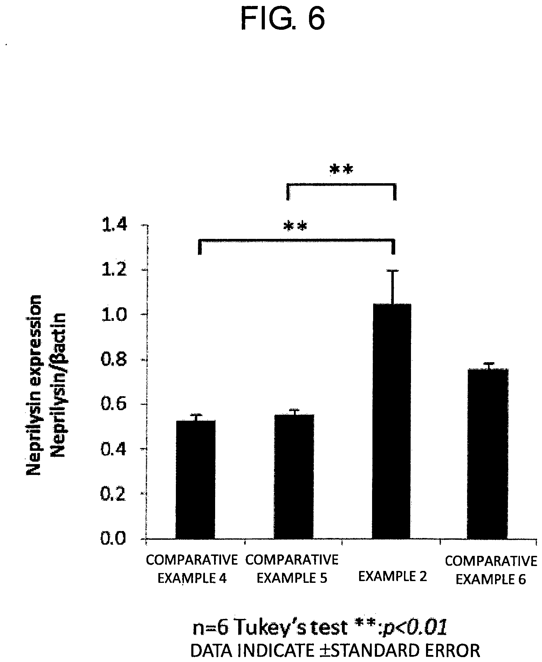

With the cDNA of the gene expression analysis sample of Example 1 as a template, the gene expression amount of a neprilysin gene (forward primer: 5'-CCAAACTTAAGCCTATTCTTAC-3': SEQ ID NO: 7 and reverse primer: 5'-CCATTATGAACCTCCAGGAC-3': SEQ ID NO: 8) was measured by the real-time PCR method using Sso Advanced™ Universal SYBR (registered trademark) Green Supermix (Bio-Rad Laboratories, Inc.) and C1000™ Thermal Cycler (CFX96™ Optics Module, Bio-Rad Laboratories, Inc.). As an internal standard, a β-actin gene (forward primer: 5'-CATCCGTAAAGACCTCTATGCCAAC-3': SEQ ID NO: 3 and reverse primer: 5'-ATGGAGCCACCGATCCACA-3': SEQ ID NO: 4) was used. FIG. 6 illustrates the results. The presence or absence of a significant difference was determined by a Tukey's test, the results of which are illustrated in FIG. 6. Neprilysin is a peptidase and is reported to decompose β-amyloid. It is also reported that the accumulation of β-amyloid and the progress of the amyloid condition of Alzheimer's disease are related to a reduction in a neprilysin level in the brain. A larger neprilysin expression amount further lessens the accumulation of β-amyloid and exhibits a higher effect of lessening Alzheimer-type dementia.

### [Comparative Example 4]

Using the gene expression analysis sample of Comparative Example 1, expression analysis on the neprilysin gene was conducted similarly to Example 2. FIG. 6 illustrates the results.

### [Comparative Example 5]

Using the gene expression analysis sample of Comparative Example 2, expression analysis on the neprilysin gene was conducted similarly to Example 2. FIG. 6 illustrates the results.

### [Comparative Example 6]

Using the gene expression analysis sample of Comparative Example 3, expression analysis on the neprilysin gene was conducted similarly to Example 2. FIG. 6 illustrates the results.

### [Example 3]

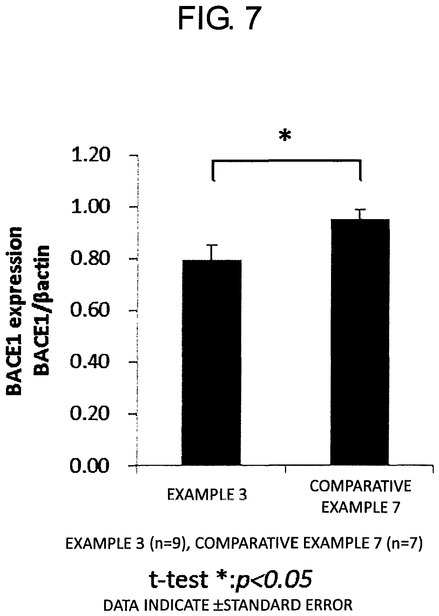

With a cDNA of a gene expression analysis sample obtained similarly to Example 1 except that nine mice were used as a template, expression analysis on the BACE1 gene was conducted similarly to Example 1. FIG. 7 illustrates the results. The presence or absence of a significant difference was determined by a t-test, the results of which are illustrated in FIG. 7.

### [Comparative Example 7]

Using a gene expression analysis sample obtained similarly to Comparative Example 3 except that seven mice were used, expression analysis on the BACE1 gene was conducted similarly to Example 1. FIG. 7 illustrates the results.

### [Example 4]

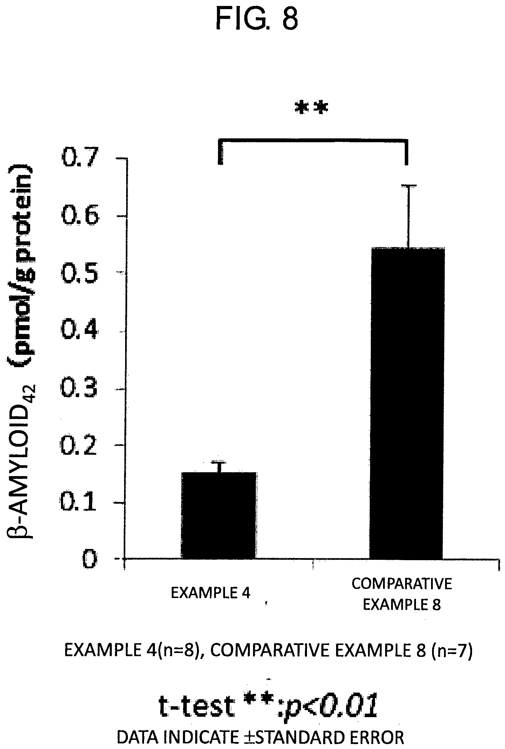

Using a protein analysis sample obtained similarly to Example 1 except that eight mice were used, total proteins and β-amyloid 42 were quantified similarly to Example 1. FIG. 8 illustrates the results. The presence or absence of a significant difference was determined by a t-test, the results of which are illustrated in FIG. 8.

### [Comparative Example 8]

Using the seven mice of Comparative Example 3, total proteins and β-amyloid 42 were quantified similarly to Example 1. FIG. 8 illustrates the results.

As illustrated in FIG. 6, the mice of Example 2, which had ingested the Sunrouge tea leaf extract, were significantly higher in the expression amount of the neprilysin gene than the normal mice of Comparative Example 4 and the mice of Comparative Example 5, which had not ingested the tea leaf extract. This indicates that the agent of the present invention effectively promotes the expression of the neprilysin gene and also indicates that the agent of the present invention can promote the decomposition of β-amyloid.

As illustrated in FIG. 7, the mice of Example 3, which had ingested the Sunrouge tea leaf extract, significantly reduced in the expression amount of the BACE1 gene compared with the mice of Comparative Example 7, which had ingested the Yabukita tea leaf extract. This indicates that the fact that the agent of the present invention effectively lessens the expression of the BACE1 gene is caused by the composition unique to the agent of the present invention.

As illustrated in FIG. 8, the mice of Example 4, which had ingested the Sunrouge tea leaf extract, were significantly lower in the accumulation amount of β-amyloid 42 (Aβ42) than the mice of Comparative Example 8, which had ingested the Yabukita tea leaf extract. This indicates that the fact that the agent of the present invention effectively lessens the accumulation of β-amyloid is caused by the composition unique to the agent of the present invention.

The results of the examples indicate that the agent of the present invention regulates the expression of the neprilysin gene to promote the decomposition of β-amyloid, can thereby protect the brain function, can protect cranial nerves, and can prevent or alleviate Alzheimer-type dementia and also indicate that the action to promote the expression of the neprilysin gene, the action to promote the decomposition of β-amyloid, the action to lessen the expression of the BACE1 gene, and the action to lessen the accumulation of β-amyloid 42 (Aβ42) of the agent of the present invention are caused by the characteristic composition of the agent of the present invention, which is different from the Yabukita tea leaf extract.

The following shows compounding examples of the agent of the present invention. The compounding examples are by way of example; the Sunrouge tea leave extract may be replaced with a composition containing another component of the plant body of the hybrid between Camellia sinensis and Camellia taliensis, for example.

### Compounding Example 1 Tablet

### (Prescription) (%)

(1) Lactose 24.0%
(2) Crystalline cellulose 20.0%
(3) Cornstarch 15.0%
(4) Dextrin balance
(5) Glycerin fatty acid ester 5.0%
(6) Sunrouge tea leaf extract 0.2%

### Compounding Example 2 Drink

### (Prescription) (%)

(1) High fructose corn syrup 30.0%
(2) Emulsifier 0.5%
(3) Sunrouge tea leaf extract 0.2%
(4) Flavoring proper amount
(5) Purified water balance

### Compounding Example 3 Jelly

### (Prescription) (%)

(1) Reduced syrup 40.0%
(2) Granulated sugar 20.0%
(3) Glucose 20.0%
(4) Gelatin 3.0%
(5) Sunrouge tea leaf extract 0.2%
(6) Purified water balance

### [Sequence Listing Free Text]

SEQ ID NO: 1: BACE1 forward primer
SEQ ID NO: 2: BACE1 reverse primer
SEQ ID NO: 3: β-actin forward primer
SEQ ID NO: 4: β-actin reverse primer
SEQ ID NO: 5: BCL-2 forward primer
SEQ ID NO: 6: BCL-2 reverse primer
SEQ ID NO: 7: Neprilysin forward primer
SEQ ID NO: 8: Neprilysin reverse primer

## Claims

1. A brain function-protecting agent comprising a component of a plant body of a hybrid between Camellia sinensis and Camellia taliensis as an active component.

2. The agent according to claim 1, wherein the agent is for alleviating a reduction in cognitive ability, preventing or alleviating dementia, or cranial nerve protection.

3. The agent according to claim 1 or 2, wherein the agent regulates one or more selected from the group consisting of accumulation of β-amyloid, BACE expression, BCL expression, and neprilysin expression.

4. The agent according to claim 2 or 3, wherein the dementia is Alzheimer-type dementia.

5. The agent according to any one of claims 1 to 4, wherein the hybrid between Camellia sinensis and Camellia taliensis is Sunrouge.

6. The agent according to any one of claims 1 to 5 comprising a tea leaf extract of the plant body of the hybrid between Camellia sinensis and Camellia taliensis.

7. A method for protecting a brain function of an object, the method comprising administering an effective amount of an agent containing a component of a plant body of a hybrid between Camellia sinensis and Camellia taliensis as an active component to the object.

8. Use of a component of a plant body of a hybrid between Camellia sinensis and Camellia taliensis in manufacturing a brain function-protecting agent.
